# EUROPEAN PATENT SPECIFICATION

(11) **EP 2 591 088 B1**
(45) Date of publication and mention of the grant of the patent: **21.04.2021**
(21) Application number: 11803898.3
(22) Date of filing: 28.06.2011
(51) Int. Cl.: C12M 1/36, C12M 1/107, C12M 1/34

(54) **SYSTEM FOR BIOLOGICAL FERMENTATION**
SYSTEM ZUR BIOLOGISCHEN FERMENTIERUNG
SYSTÈME DE FERMENTATION BIOLOGIQUE

(30) Priority: 05.07.2010 US 361451 P; 05.07.2010 SE 1050738
(43) Date of publication of application: 15.05.2013
(73) Proprietor: BPC Instruments AB, 223 63 Lund (SE)
(72) Inventor: LIU, Jing, S-226 48 Lund (SE)
(74) Representative: AWA Sweden AB
(86) International application number: PCT/SE2011/050860
(87) International publication number: WO 2012/005667

(56) References cited:
- EP-A1- 0 947 821
- EP-A2- 0 263 634
- EP-A2- 0 263 634
- WO-A1-2007/044699
- CN-Y- 201 062 268
- FR-A1- 2 455 629
- GB-A- 2 158 840
- JP-A- 2007 312 676
- US-A- 3 926 738
- US-A- 3 926 738
- US-A- 6 029 101
- US-A1- 2005 272 146
- US-A1- 2006 001 865
- US-A1- 2009 215 176
- ENFORS ET AL. BIOPROCESS TECHNOLOGY - FUNDAMENTALS AND APPLICATIONS 2000, page 30, XP008159854

## Description

### Field of invention

The present invention relates to a system for biological fermentation, in particular to a system of lab-scale size or small pilot scale size for simulation of biological fermentation. Moreover, the present invention also concerns a method for controlling a biological fermentation process system.

### Technical Background

Different fermentors (or bioreactors) and process system therefore used in biological fermentation processes for producing biogas are known. One such example is described in DE10306988A, which discloses a fermentor for biogas that is rotated to supply natural mixing and continuous movement of the biomass. Continuous feeding and discharge is said to be achieved according to DE10306988A.

Moreover, in GB2158840A there is disclosed an apparatus for continuous fermentation or gas production, comprising a series of connected tanks using non-returnable ball valves, that is check valves, to create a continuous one-way flow of fluids and gas, main or sole operating force being the gas pressure produced within the apparatus. The tanks may have heating coils or pipes connected to solar panels. The fermentation process can be applied to brewing, wine and alcohol production; or any liquid gas producing reaction. Gas pressure is used to perform functions normally done by labour, machines and pumps. As may be understood from above, GB2158840A is directed to using "self-produced" pressure obtained from the fermentation to perform mechanical work and thus create a continuous one-way flow of fluids and gas.

One drawback of the apparatus disclosed in GB2158840A is the controlling of the different tanks and the non-existing means to analyse the fermentation process as a whole in terms of e.g. yield of gas production. Moreover, another drawback is the actual controlling of the fermentation process, which is only based on creating a continuous one-way flow of fluids and gas by using the gas pressure produced within the apparatus.

Furthermore, in EP0263634 there is disclosed a culture system in which a culture medium is supplied to a culture vessel by providing pressure difference of a gas by means of which the culture medium is driven into the culture vessel.

US3926738 discloses an apparatus for control of biochemical processes, comprising vessel means within which said process is to be performed; means for adding controlled amounts of substances required for said process into said vessel means; means for monitoring a plurality of predetermined controllable and dependent variables in said process; calculating means for calculating at least one further, not directly measurable dependent variable representative of the status of the biochemical process on a real-time, on-line basis from said detected controllable and dependent variables; and control means for interactively regulating the values of said controllable variables in response to said calculated values of said further dependent variable to bring said further dependent variable to predetermined levels to create the desired environmental conditions for the biochemical process.

One aim of the present invention is to provide a system for biological fermentation, which system renders high control of the fermentation process.

Another purpose of the present invention is to provide a system which ensures high accuracy in relation to feeding and discharging for control of a fermentor or a fermentation process.

### Summary of invention

The stated purposes above are achieved by a system for biological fermentation, said system comprising:
- a feedstock tank connected to a bioreactor, wherein the bioreactor has a slurry/liquid inflow, a slurry/liquid outflow and a gas outflow;
- a feeding valve to control the feeding and hence slurry/liquid inflow from the feedstock tank to the bioreactor;
- a computer unit which controls the opening and closing of the feeding valve, wherein the feeding from the feedstock tank to the bioreactor is driven by a higher pressure in the feedstock tank than in the bioreactor; and wherein mass balance control is ensured by control of opening and closing of the feeding valve in relation to a set point value of the mass quantity on the computer unit and wherein the system also comprises
- a waste receiving tank; and
- a discharging valve and/or a discharging pump to control the discharging of slurry/liquid waste and hence slurry/liquid outflow from the bioreactor to the waste receiving tank,
wherein the computer unit also controls the opening and closing of the discharging valve and/or the controlling of the discharging pump in relation to a set point value of the mass quantity on the computer unit, and wherein the discharging from the bioreactor to the waste receiving tank is driven by a higher pressure in the bioreactor than in the waste receiving tank or directly by controlling the discharging pump and the optional discharging valve.

In EP0263634, there is disclosed a system where pressure difference is used as a driving force for moving a liquid flow. However, the present invention differs from the system disclosed in EP0263634 on several important aspects. Firstly, the system according to the present invention may achieve not only creating a flow of feedstock/slurry but also good mass control, which is achieved by the combination of pressure difference plus computer controlled mass control. EP0263634 only refers to moving a liquid by pressure difference, and there is no information at all about mass balance control and how this may be achieved.

Secondly, in EP0263634 the mass is controlled by changing the pressure difference (see page 3, line 31-32). According to the present invention, pressure difference is only the driving force and the mass is controlled by balance separately. This renders a far more reliable approach since the flow rate of liquid or slurry does not only depend on the pressure difference but also the propriety of the liquid and slurry. If the character of liquid and slurry varies, the flow will change as well even under the same pressure difference. Furthermore, there is possibility that flow will change over a long time operation. If the mass is calculated by the fix flow rate, over- or under-estimation may occur. According to the present invention, mass is controlled by balance which is independent from the flow rate, and therefore is much more reliable.

Finally, the purpose of EP0263634 is totally different. EP0263634 intends to solve the problem of frequent tube damage, contamination and pulsation, e.g. if a peristaltic pump is used. To keep mass balance control is not even addressed in EP0263634, which is a key matter of the present invention.

According to the present invention, a digital balance is used to together with the same computer that is used to create pressure difference for driving the feedstock and opening/closing valves for directing the flow of feedstock and slurry. This is very different from the approach disclosed in EP0263634.

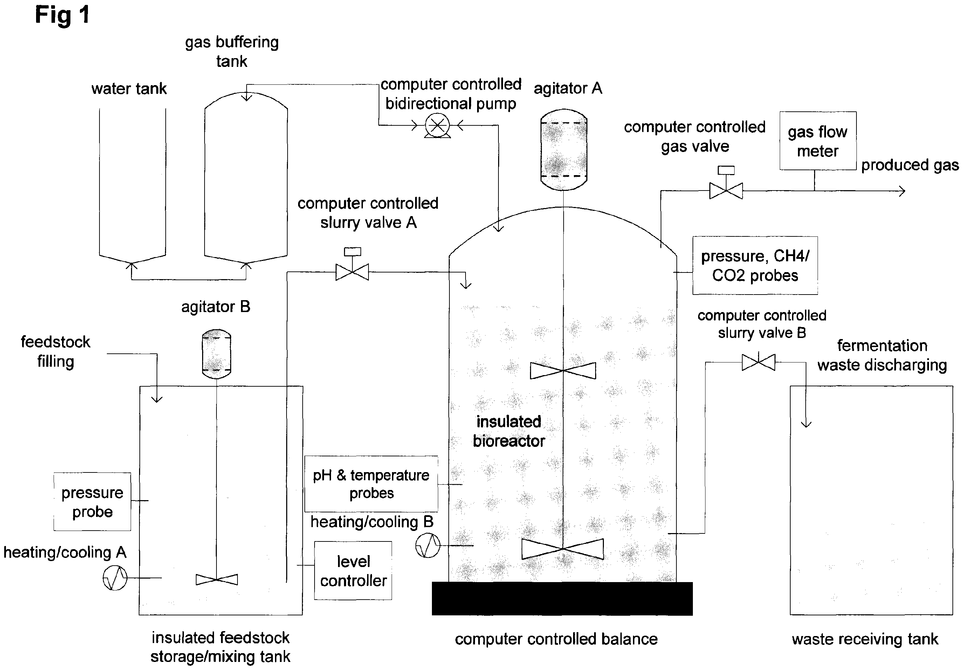

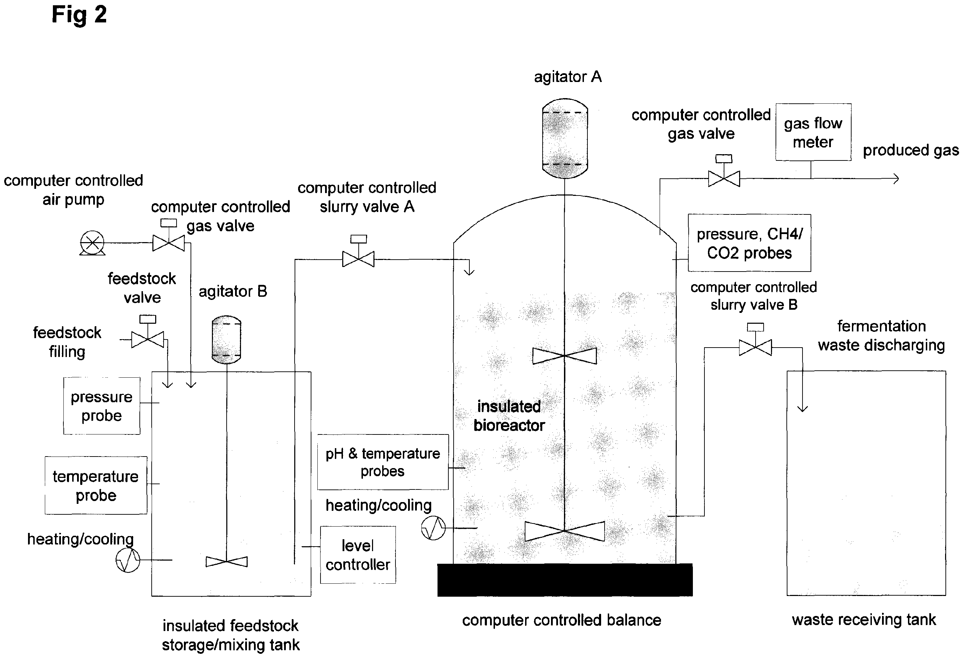

Moreover, as may be noted from above, the design of the system according to the present invention differs from the apparatus disclosed in GB2158840A. In GB2158840A there is disclosed a series of tanks wherein a continuous one-way flow of fluids and gas is to be achieved by the pressure build-up in the apparatus. This is not utilized in the system according to the present invention, which may be seen from the system examples of the present invention shown in fig. 1 and 2 and described below. Moreover, according to the present invention a computer unit is used to control the opening and closing of the feeding valve on the piping between the feedstock tank and the bioreactor. According to GB2158840A, the valves, although having different purposes, are controlled, directly or indirectly, by the gas pressure in the process, and not by a computer unit.

### Brief description of the drawings

Fig. 1 shows a system setup according to one embodiment of the present invention.
Fig. 2 shows a system setup according to another specific embodiment of the present invention.

### Background to process simulation experiments for biological fermentation

Today, lab-scale or small pilot-scale process simulation experiment for biological fermentation is often conducted manually, which is very labour intensive and time consuming process over a period of a couple of months even over one year. This kind of experiment also requires skilled engineers to carry out the work. Moreover, the results of such simulation experience studies are often poor due to problem of feeding, unsatisfactory data quality in terms of measurement errors, low sampling intervals, non-suitable working schedule, etc. Main challenges of continuous process simulation experiment include:
- Problem of not being able to simulate continuous feeding due to problem of pump clogging. This is a typical problem for high solid content feedstock, such as slurry like substrates. It is hardly to find a suitable pump that is commercially available for handling small flow of feedstock without clogging problem.
- Even if certain pumps may work for a short period of time for low solid content feedstock, it is still very hard to simulate continuous feeding due to the reason that the pumping capacity of most pumps is too high. For example, if one needs to run a 2 litres digestion with solid retention time of 25 days, only 80 ml per day needs to be pumped into the digester/fermentor/- bioreactor. It is extremely difficult to pump this small volume continuously and precisely over 24 hours. Instead, the normal methods is to inject 80 ml at one time, which is not considered as continuous feeding, and it is therefore not possible to simulate the real continuous operation in full-scale plants.
- The reason to carry out continuous process experiment is often to simulate the full-scale operation for the purpose of process optimization. In order to truly simulate the full-scale process, on-line monitoring of process parameters, such as biogas flow, biogas composition, pH, etc. are desire, in particular for biogas flow. Due to the fact that the biogas flow rate in bench-scale experiments is often very low, it is also hardly to find suitable flow meter that is commercially available today to meet the monitoring requirement for ultra low gas flow. - Mass balance analysis is an important tool for developing the optimization tool. However, data from the traditional lab-scale or small pilot-scale process simulation cannot meet the requirements for the mass balance analysis due to the long data sampling time interval (too less data) and poor data quality.

### Specific embodiments of the invention

Specific embodiments of the present invention are described below.

According to one embodiment, the content of the bioreactor is continuously weighted. This may give an advantage when keeping mass balance control on a regular basis and not only checked at specific time points in the computer unit.

As mentioned above, according to the present invention the system also comprises
- a waste receiving tank; and
- a discharging valve and/or a discharging pump to control the discharging of slurry/liquid waste and hence slurry/liquid outflow from the bioreactor to the waste receiving tank,
wherein the computer unit also controls the opening and closing of the discharging valve and/or the controlling of the discharging pump in relation to a set point value of the mass quantity on the computer unit, and wherein the discharging from the bioreactor to the waste receiving tank is driven by a higher pressure in the bioreactor than in the waste receiving tank or directly by controlling the discharging pump and an optional discharging valve.

As may be noted from above, the computer unit may control both the feeding valve and the discharging valve, and e.g. a feeding pump if present. Moreover, the pressure difference or a discharging pump is the driving force also in this case when discharging shall be made from the bioreactor to the waste receiving tank.

As may be seen in the figures, the feeding valve is denoted as a computer controlled slurry valve A, and the discharging valve is denoted as a computer controlled slurry valve B.

It is important to realize that the system according to the present invention works for both material in slurry form and liquid form. However, as the processes available today inter alia have problems with pump clogging when the material is in slurry form, the present invention solves this specific problem very well.

Another purpose of the present invention is to provide a system which allows for real-time analysis to be made during the fermentation. Such a system may be operated in full scale production, but the system according to the present invention finds specific use for lab-scale or pilot scale for simulation and analysis of a fermentation process. By solving the problems disclosed above for lab-scale or small pilot-scale process simulation experiments of today, the system according to the present invention is very suitable for such simulation of a fermentation process.

According to one specific embodiment of the present invention, the high precision of mass quantity control for feeding and discharging is achieved by a computer controlled balance. The content of the bioreactor is continuously weighted and the mass balance control is ensured by control of opening and closing of the feeding valve and discharging valve with the coordination of opening and closing gas valve and actuating the bidirectional pump (in case of example illustrated in fig.1) or opening and closing gas valve A & B and actuating the computer controlled air pump (in case of example illustrated in fig. 2). By continuously weighing the content of the bioreactor and record that on the computer unit, it is according to the present invention possible to use a computer controlled mass balance to control the feeding and discharging into and from the bioreactor. If the weight of the content of the bioreactor alters, the computer unit sends a signal to the feeding valve or discharging valve to open, and thereby keeping the same mass inside of the bioreactor. By the present invention, the amount of feeding and discharging can be very well controlled by the computer controlled balance. Therefore a very accurate pumping and discharging can be carried out by combining the "pressure driven" feeding/discharging approach and balance controlled feeding and discharging valves. For lab- and bench-scale, balance should be the right choice. However, if the technical approach according to the present invention is applied to pilot- or larger scale, balance in right size might not be available anymore. Instead, a suitable slurry or sludge mass flow meter might be used to control the mass throughput. It is important to realize that also such a solution is possible according to the present invention.

As may be understood from above, according to one embodiment of the present invention, the system also comprises a computer unit controlled gas valve and a gas flow meter for the gas outflow. According to the system setups disclosed in the figures, the gas valve connected to the bioreactor is used for assisting in the creation of positive (over-) or negative (under-) pressure, whereas the gas flow meter is used for monitoring the gas outflow. Preferably, also the gas flow meter is connected to the computer unit so that the computer unit may record the actual biogas production. Furthermore, according to one specific embodiment of the present invention, the measuring principle of the gas flow meter is preferably based on the liquid displacement gas flow meter. Such gas measuring devices find specific use for ultra low gas flows, and may as such be suitable to use for e.g. system setups of lab- and bench-scale size according to the present invention. One example of an appropriate liquid displacement gas flow meter for the system according to the present invention is described in the international patent application PCT/SE2010/050371. It is however important to realize that any gas flow meter with proper measuring accuracy and range may be used according to the present invention, and not only gas flow meters working by liquid displacement.

According to the present invention, the pressure difference and thus driving force may be achieved in different ways. According to one specific embodiment of the present invention, the system also comprises a computer unit controlled bidirectional pump intended to pump gas in or out of the bioreactor in order to create a negative pressure for sucking the feedstock from the feedstock tank to the bioreactor or to create a positive pressure for discharging slurry/liquid waste from the bioreactor to the waste receiving tank. This approach according to the present invention is shown in fig. 1. The gas used may either be inert gas, e.g. N₂, or could be gas produced during the fermentation process. According to another specific embodiment of this approach according to the present invention, the system also comprises a gas buffering tank and a water tank which provide gas storage for pumping gas to/from the bioreactor. This is also shown in fig. 1.

According to another specific embodiment of the present invention, which approach is shown in fig. 2, the system also comprises a computer unit controlled pump intended to pump gas to the feedstock tank in order to create a positive pressure in the feedstock tank. This approach may be used to drive the slurry/liquid feedstock from the feedstock tank to flow into the bioreactor. The discharging from the fermentation broth is possible once the over pressure is created due to the accumulation of gases produced during fermentation process. In this case, the gas pumped in may e.g. be air or N₂.

As hinted above, the system according to the present invention may comprise a discharging pump which is computer-controlled to be able to keep mass balance control. This is an alternative instead of a pressure driving force on the discharging side. Such a pump may in fact also be integrated with feeding performed with pressure difference as driving force and valves controlled by the computer unit.

The system according to the present invention may also comprise additional components. According to one embodiment, the feedstock tank and/or the bioreactor each comprise at least one heating/cooling system and/or at least one agitator. As shown in fig. 1 and 2, both the feedstock tank and the bioreactor comprise agitators and heating/cooling systems. Different such agitators and heating/cooling systems may be used.

According to another specific embodiment of the present invention, the feedstock tank may comprise at least one from the group consisting of a level controller, a temperature probe and a pressure probe. Several such controllers and probes may used. Moreover, for example a valve for the piping for feedstock filling into the feedstock storage/mixing tank may also be provided and may also be computer controlled, but this is not a must.

According to one embodiment of the present invention, the bioreactor may comprise at least one from the group consisting of a pH probe, a temperature probe, a pressure probe, a CO₂ probe and a CH₄ probe. Also in this case all of these probes may be provided, and e.g. multiple probes of each type for detecting value profiles of target parameters and/or security reasons may also be provided if that is preferred.

It is important to understand that all of these additional components may suitably be connected to the computer unit so that the values of e.g. pressure, temperature, levels etc. may be registered on the computer unit.

As may be understood from above, the system according to the present invention may be used for large scale production, however the system setup is especially intended for small size, such as lab-scale or pilot scale, e.g. for a fermentor/bioreactor size of a few litres upwards.

According to the present invention, there is also disclosed a method for controlling a biological fermentation process system according to the invention, wherein the method comprises using a computer unit by means of
- receiving data on the weight of the content in the bioreactor;
- upholding mass balance in the bioreactor by controlling the opening and closing of the feeding valve and discharging valve in relation to a set point value of the mass quantity on the computer unit.

According to one specific embodiment of the present invention, said method comprises a bioreactor filling sequence, which is achieved by creating a pressure difference between the feedstock tank and the bioreactor so that there is a higher pressure in the feedstock tank than in the bioreactor and thereby creating a pressure driving force from the feedstock tank to the bioreactor.

According to another specific embodiment of the present invention, said method comprises a bioreactor discharging sequence, which is achieved by creating a pressure difference between the bioreactor and the waste receiving tank so that there is a higher pressure in the bioreactor than in the waste receiving tank and thereby creating a pressure driving force from the bioreactor to the waste receiving tank.

According to one embodiment of the present invention, upholding mass balance in the bioreactor is achieved by the controlling of the opening and closing of the feeding valve and discharging valve with the coordination of the opening and closing of a computer unit controlled gas valve for the gas outflow from the bioreactor.

According to one specific embodiment, the upholding of the mass balance in the bioreactor is additionally achieved by actuating a bidirectional pump intended to pump gas in or out of the bioreactor in order to create a negative pressure for sucking the feedstock from the feedstock tank to the bioreactor or to create a positive pressure for discharging slurry/liquid waste from the bioreactor to the waste receiving tank.

According to another specific embodiment, the upholding of the mass balance in the bioreactor is additionally achieved by actuating a pump intended to pump gas to the feedstock tank in order to create a positive pressure in the feedstock tank.

According to yet another specific embodiment, said method also comprises online registration of the gas flow on the computer unit by means of the gas flow meter. It is important to understand that not only the gas flow meter may be connected to the computer unit, but also other sensors/probes/- detectors may of course be connected to the computer unit for online registration, such as detectors for pH, temperature, pressure, gas composition, slurry level etc.

### Detailed description of the drawings

The basic principle for the present invention is to utilize positive (over-) and negative (under-) pressure created on purpose to drive the substrate/- feedstock feeding and sludge/waste discharging, and use a digital balance for ensuring the accurate mass control. Two examples of system designs based on the present invention can be found in the figures.

With reference to fig. 1, this figure shows a system setup according to one specific embodiment of the present invention. As may be seen, the system may comprise an insulated bioreactor which is provided with an agitator, online probes (pH, temperature, pressure, CH₄, CO₂) and a heating/cooling system for both options of heating and/or cooling. The bioreactor is placed on a weighing machine or balance with a desired set point to enable to uphold computer controlled mass balance by use of the computer unit (not shown) and suitable software. The bioreactor is provided with piping for the produced biogas (CH₄ together with CO₂, but also possibly small amounts of other gases, like H₂S, H₂). This biogas production piping is provided with a gas valve as well as a gas flow meter for ensuring substrate feeding and sludge discharging, as well as measuring the amount of produced gas. The gas flow meter is connected to the computer unit so that the amount of gas may be registered. The produced biogas is e.g. fed to a gas storage tank.

The system also comprises an insulated feedstock storage/mixing tank which may be provided with an agitator, temperature probe, level controller and a heating/cooling system. Furthermore, the system also comprises a waste receiving tank. The feedstock (slurry) tank are provided with means for filling the feedstock tank with feedstock, possibly by piping provided with a feedstock valve, and the waste receiving tank may be provided with means for discharging also that tank. Moreover, the feedstock tank may be provided with a port so that air or inert gas can flow in to replace the feedstock volume fed into the bioreactor.

The feedstock tank is connected to the bioreactor via piping provided with a computer controlled feeding valve (denoted as computer controlled slurry valve A). The bioreactor is connected to the waste receiving tank via piping provided with a discharging valve (denoted as computer controlled slurry valve B). These piping connections enable slurry to be fed from the feedstock tank into the bioreactor and from the bioreactor to the waste receiving tank. This is accomplished by use of a difference in pressure between the different containers but also by use of the computer unit which controls the opening and closing of the feeding and discharging valves with the coordination of opening and closing of the computer controlled gas valve and actuating the bidirectional pump (also computer controlled) by use of software targeted to mass balance control of the bioreactor.

The process sequence for upholding mass balance in the bioreactor is made according to the following. A desired volume/mass feeding set point is the starting point for a sequence. When this is set on the computer unit, that volume/mass is pumped into the bioreactor and then afterwards the same mass/volume is discharged from the bioreactor. However, if the sequence is run this way, there may be a problem in view of the fact that some amount of fresh feedstock, which just have been pumped into the bioreactor, may follow the waste being discharged from the bioreactor. Therefore, preferably the discharging from the bioreactor is made before the filling of the bioreactor with fresh feedstock in the sequence. During this sequence, the computer unit sends signals to the valves to open and close at the intended time points.

With reference to the system setup according to fig. 1, one more detailed example of a process sequence is described below. When there is a need to feed the bioreactor with a desired quantity feedstock from the feedstock storage tank, the computer unit sends signals to the gas valve to close, actuates the bidirectional pump to suck gas from bioreactor to the gas buffering tank, and then to the slurry valve A to open so that feedstock may be fed into the bioreactor. When the desired quantity of mass is entered into the bioreactor due to the feedstock feeding, the computer unit sends signals to the slurry valve A to close and de-actuates the bidirectional pump. Consequently, there is a need to discharge the bioreactor with the same quantity sludge from the bioreactor to the waste receiving tank. The computer unit re-actuates the bidirectional pump to inject gas from gas the buffering tank to the bioreactor, then sends a signal to the slurry valve B to open so that sludge may be discharged into the waste receiving tank. When the desired quantity of mass is discharged from the bioreactor, the computer unit sends signals to the slurry valve B to close and de-actuates the bidirectional pump. It should be noticed that the feeding and discharging sequence can be varied a lot depending on the need of the process operation. For example and as mentioned above, the discharging of the bioreactor is preferably in fact made before the actual feeding to the bioreactor even if a desired feeding of fresh feedstock into the bioreactor is the starting point for a new filling and discharging sequence. Therefore, other feeding and discharging sequences will certainly be feasible. The important feature in relation to the feeding and discharging of the bioreactor of the system according to the present invention is at all times the drive towards upholding the mass balance in the bioreactor in order to ensure a long-term continuous operation.

As may be seen and understood from above, according to the system setup shown in fig. 1, the bioreactor is connected to a computer controlled bidirectional pump. As mentioned above, the bidirectional pump is intended to pump gas in or out of the bioreactor in order to create a negative pressure for sucking the feedstock from the feedstock tank to the bioreactor (during a bioreactor filling cycle) or to create a positive pressure for discharging slurry waste from the bioreactor to the waste receiving tank (during a bioreactor discharging cycle). The bidirectional pump is connected to a gas buffering tank and a water tank which provide gas storage for pumping inert gas or gases produced during fermentation process to/from the bioreactor.

With reference to fig. 2, this figure shows another system setup according to the present invention. The only difference of this setup in comparison with the setup shown in fig. 1 is the means for creating the pressure difference between a discharging container and a receiving container. In this case the feedstock tank is connected to a computer controlled air pump (or N₂ pump) intended to create a positive pressure in the feedstock tank. This approach may be used to drive the feedstock from the feedstock tank to flow into the bioreactor. The discharging from the fermentation broth is possible once the over pressure is created due to the accumulation of gases produced during the fermentation process. In this case, the feedstock tank may also be provided with a pressure probe for measuring the gas pressure.

With reference to fig. 1 and 2 and the difference in creating the pressure driving force, for the system setup shown in fig. 1 the gas valve is closed during the feedstock feeding in order to create a negative pressure and is remain closed during the sludge discharging in order to create a positive pressure to push the sludge out of the bioreactor and into the waste receiving tank. For the system setup shown in fig. 2, the gas valve is closed during the feedstock feeding in order to create a positive pressure and is remain closed during the sludge discharging in order to utilize the positive pressure to push the sludge out of the reactor and into the waste receiving tank. Moreover, during the non-feeding and non-discharging periods, the gas valve remains open, the bidirectional pump remains closed, and the feeding valve and discharging valve remain closed.

As mentioned above, according to the present invention, there exist various operational procedures available by controlling the operational sequence of various pumps and valves. This will be implemented through the control software for the computer unit according to the present invention.

### Conclusions

The present invention provides several advantages in comparison to known solutions today and solves several problems mentioned above. The advantages of the present invention are summarised below.
- The problem of pump clogging can be easily solved using the "pressure driven" feeding approach according to the present invention. The risk for clogging will be very low since one just needs to select a suitable size of pipe for transfer the slurry-like feedstock. The driving force for pumping feedstock is based on the under pressure (system example nr. 1) or over pressure (system example nr. 2) which is created on purpose by a simple pump without direct contact of slurry feedstock. The amount of feeding and discharging can be very well controlled by a computer controlled balance. Therefore a very accurate pumping and discharging can be carried out by combining the "pressure driven" feeding approach and balance controlled slurry valves according to the present invention. For bench-scale, balance should be the preferred choice. However, if this technical approach is applied to pilot- or larger scale, balance might not be suitable anymore. Instead, a suitable slurry or sludge mass flow meter may be used to control the mass throughput. If feeding and/or discharging pumps are used according to the present invention, it is thus important to choice such pumps having minimized risk of clogging.
- Based on the current technical solution according to the present invention, a very small volume of feeding can be carried out. Based on a preliminary test, only few gram of slurry can be accurately pumped into the digester/bioreactor without any problem. Therefore, almost perfect continuous (i.e. large number of small discrete feedings, and overall it looks like a perfect continuous feeding) feeding can be carried out automatically. Similarly, the discharging of the sludge can also be carried out in the same manner.
- The flow meter and sensors (probes), such as pressure sensor, temperature sensor, CO₂ sensor, pH sensor, etc can also be included in the current (simulation) system platform according to the present invention.
- Due to the accuracy of balance, an accurate mass balance analysis can be easily carried out using the current platform. If the mass throughput can be accurately monitored using slurry flow meter in pilot- or larger scale process, a good mass balance analysis is still possible for such processes of larger sizes.

## Claims

1. System for biological fermentation, said system comprising:
- a feedstock tank connected to a bioreactor, wherein the bioreactor has a slurry/liquid inflow, a slurry/liquid outflow and a gas outflow;
- a feeding valve to control the feeding and hence slurry/liquid inflow from the feedstock tank to the bioreactor;
- a computer unit which controls the opening and closing of the feeding valve, wherein the feeding from the feedstock tank to the bioreactor is driven by a higher pressure in the feedstock tank than in the bioreactor; and
wherein mass balance control is ensured by control of opening and closing of the feeding valve in relation to a set point value of the mass quantity on the computer unit and wherein the system also comprises
- a waste receiving tank; and
- a discharging valve and/or a discharging pump to control the discharging of slurry/liquid waste and hence slurry/liquid outflow from the bioreactor to the waste receiving tank,
wherein the computer unit also controls the opening and closing of the discharging valve and/or the controlling of the discharging pump in relation to a set point value of the mass quantity on the computer unit, and wherein
the discharging from the bioreactor to the waste receiving tank is driven by a higher pressure in the bioreactor than in the waste receiving tank or directly by controlling the discharging pump and the optional discharging valve.

2. System according to claim 1, wherein the content of the bioreactor is continuously weighted.

3. System according to claim 1 or 2, wherein the system also comprises a computer unit controlled gas valve and a gas flow meter for the gas outflow.

4. System according to claim 3, wherein the gas flow meter is a liquid displacement gas flow meter.

5. System according to any one of claims 1-4, wherein the system also comprises a computer unit controlled bidirectional pump intended to pump gas in or out of the bioreactor in order to create a negative pressure for sucking the feedstock from the feedstock tank to the bioreactor or to create a positive pressure for discharging slurry/liquid waste from the bioreactor to the waste receiving tank.

6. System according to claim 5, wherein the system also comprises a gas buffering tank and a water tank which provide gas storage for pumping gas to/from the bioreactor.

7. System according to any one of claims 1-6, wherein the system also comprises a computer unit controlled pump intended to pump gas to the feedstock tank in order to create a positive pressure in the feedstock tank.

8. System according to any one of the preceding claims, wherein the feedstock tank and/or the bioreactor each comprise at least one heating/cooling system and/or at least one agitator.

9. System according to any one of the preceding claims, wherein the feedstock tank comprises at least one from the group consisting of a level controller, a temperature probe and a pressure probe.

10. System according to any one of the preceding claims, wherein the bioreactor comprises at least one from the group consisting of a pH probe, a temperature probe, a pressure probe, a CO₂ probe and a CH₄ probe.

11. System according to any one of the preceding claims, wherein the system is of lab-scale size or small pilot scale size.

12. Method for controlling a biological fermentation process system according to any of the preceding claims, said method comprising using a computer unit by means of
- receiving data on the weight of the content in the bioreactor;
- upholding mass balance in the bioreactor by controlling the opening and closing of the feeding valve and the discharging valve in relation to a set point value of the mass quantity on the computer unit.

## Patentansprüche

1. System zur biologischen Fermentation, wobei das System umfasst:
- einen Ausgangsmaterialtank, der mit einem Bioreaktor verbunden ist, wobei der Bioreaktor einen Schlamm-/Flüssigkeitszufluss, einen Schlamm-/Flüssigkeitsabfluss und einen Gasauslass aufweist;
- ein Einspeiseventil zum Steuern der Einspeisung und damit des Schlamm-/Flüssigkeitszuflusses aus dem Ausgangsmaterialtank in den Bioreaktor;
- eine Computereinheit, die das Öffnen und das Schließen des Einspeiseventils steuert, wobei die Einspeisung aus dem Ausgangsmaterialtank in den Bioreaktor durch einen höheren Druck im Ausgangsmaterialtank als im Bioreaktor angetrieben wird; und
wobei Massenbilanzsteuerung durch Steuerung des Öffnens und des Schließens des Einspeiseventils in Abhängigkeit von einem Sollwert der Massenmenge an der Computereinheit gewährleistet wird, und wobei das System außerdem umfasst:
- einen Abfallauffangbehälter; und
- ein Entleerungsventil und/oder eine Entleerungspumpe zum Steuern des Entleerens von Schlamm- /Flüssigkeitsabfall und damit von Schlamm- /Flüssigkeitsabfluss aus dem Bioreaktor in den Abfallauffangbehälter,
wobei die Computereinheit außerdem das Öffnen und das Schließen des Entleerungsventils und/oder das Steuern der Entleerungspumpe in Abhängigkeit von einem Sollwert der Massenmenge an der Computereinheit steuert und wobei die Entleerung aus dem Bioreaktor in den Abfallauffangbehälter durch einen höheren Druck im Bioreaktor als im Abfallauffangbehälter oder direkt durch Steuerung der Entleerungspumpe und des optionalen Entleerungsventils angetrieben wird.

2. System nach Anspruch 1, wobei der Inhalt des Bioreaktors fortlaufend gewogen wird.

3. System nach Anspruch 1 oder 2, wobei das System außerdem ein von einer Computereinheit gesteuertes Gasventil und einen Gasdurchflussmesser für den Gasauslass umfasst.

4. System nach Anspruch 3, wobei der Gasdurchflussmesser ein Flüssigkeitsverdrängungsgasdurchflussmesser ist.

5. System nach einem der Ansprüche 1 bis 4, wobei das System außerdem eine von einer Computereinheit gesteuerte bidirektionale Pumpe umfasst, die dazu bestimmt ist, Gas in den Bioreaktor oder aus dem Bioreaktor zu dem Zweck zu pumpen, einen Unterdruck zum Ansaugen des Ausgangsmaterials aus dem Ausgangsmaterialtank in den Bioreaktor oder einen Überdruck zum Abführen von Schlamm- /Flüssigkeitssabfall aus dem Bioreaktor in den Abfallauffangbehälter zu erzeugen.

6. System nach Anspruch 5, wobei das System außerdem einen Gaspuffertank und einen Wassertank umfasst, die Gasspeicher zum Pumpen von Gas zum/vom Bioreaktor bereitstellen.

7. System nach einem der Ansprüche 1 bis 6, wobei das System außerdem eine von einer Computereinheit gesteuerte Pumpe umfasst, die dazu bestimmt ist, Gas in den Ausgangsmaterialtank zum Erzeugen eines Überdrucks im Ausgangsmaterialtank zu pumpen.

8. System nach einem der vorhergehenden Ansprüche, wobei der Ausgangsmaterialtank und/oder der Bioreaktor jeweils mindestens ein Heiz-/Kühlsystem und/oder mindestens ein Rührwerk umfassen.

9. System nach einem der vorhergehenden Ansprüche, wobei der Ausgangsmaterialtank mindestens eines aus der Gruppe bestehend aus einer Füllstandssteuerung, einer Temperatursonde und einer Drucksonde umfasst.

10. System nach einem der vorhergehenden Ansprüche, wobei der Bioreaktor mindestens eines aus der Gruppe bestehend aus einer pH-Sonde, einer Temperatursonde, einer Drucksonde, einer CO₂-Sonde und einer CH₄-Sonde umfasst.

11. System nach einem der vorhergehenden Ansprüche, wobei das System eine Größe im Labormaßstab oder im kleinen Pilotmaßstab aufweist.

12. Verfahren zum Steuern eines Prozesssystems zur biologischen Fermentation nach einem der vorhergehenden Ansprüche, wobei das Verfahren die Verwendung einer Computereinheit zu folgenden Zwecken umfasst:
- Empfangen von Daten über das Gewicht des Inhalts im Bioreaktor;
- Aufrechterhalten von Massenbilanz im Bioreaktor durch Steuerung des Öffnens und des Schließens des Einspeiseventils und des Entleerungsventils in Abhängigkeit von einem Sollwert der Massenmenge an der Computereinheit.

## Revendications

1. Système de fermentation biologique, ledit système comprenant :
- un réservoir de matière première relié à un bioréacteur, le bioréacteur ayant une entrée de boue/liquide, une sortie de boue/liquide et une sortie de gaz ;
- une vanne d'alimentation pour commander l'alimentation et donc l'entrée de boue/liquide depuis le réservoir de matière première vers le bioréacteur ;
- une unité informatique qui commande l'ouverture et la fermeture de la vanne d'alimentation, l'alimentation du réservoir de matière première au bioréacteur étant entraînée par une pression plus élevée dans le réservoir de matière première que dans le bioréacteur ; et la commande du bilan massique étant assurée par la commande de l'ouverture et de la fermeture de la vanne d'alimentation en fonction d'une valeur de point de consigne de la quantité de masse sur l'unité informatique et le système comprenant également
- un réservoir de réception des déchets ; et
- une vanne de décharge et/ou une pompe de décharge pour commander la décharge des déchets de boue/liquide et donc l'écoulement de boue/liquide du bioréacteur vers le réservoir de réception des déchets,
l'unité informatique commandant également l'ouverture et la fermeture de la vanne de décharge et/ou la commande de la pompe de décharge en fonction d'une valeur de point de consigne de la quantité de masse sur l'unité informatique, et la décharge du bioréacteur vers le réservoir de réception des déchets étant entraînée par une pression plus élevée dans le bioréacteur que dans le réservoir de réception des déchets ou directement par la commande de la pompe de décharge et de la vanne de décharge optionnelle.

2. Système selon la revendication 1, le contenu du bioréacteur étant pesé en continu.

3. Système selon la revendication 1 ou 2, le système comprenant également une vanne de gaz commandée par une unité informatique et un débitmètre de gaz pour la sortie de gaz.

4. Système selon la revendication 3, le débitmètre de gaz étant un débitmètre de gaz à déplacement de liquide.

5. Système selon l'une quelconque des revendications 1 à 4, le système comprenant également une pompe bidirectionnelle commandée par une unité informatique destinée à pomper du gaz dans ou hors du bioréacteur afin de créer une pression négative pour aspirer la matière première du réservoir de matière première vers le bioréacteur, ou de créer une pression positive pour décharger les déchets de boue/liquide du bioréacteur vers le réservoir de réception des déchets.

6. Système selon la revendication 5, le système comprenant également un réservoir tampon de gaz et un réservoir d'eau qui fournissent un stockage de gaz pour pomper le gaz vers/depuis le bioréacteur.

7. Système selon l'une quelconque des revendications 1 à 6, le système comprenant également une pompe commandée par une unité informatique destinée à pomper du gaz vers le réservoir de matière première afin de créer une pression positive dans le réservoir de matière première.

8. Système selon l'une quelconque des revendications précédentes, le réservoir de matière première et/ou le bioréacteur comprenant chacun au moins un système de chauffage/refroidissement et/ou au moins un agitateur.

9. Système selon l'une quelconque des revendications précédentes, le réservoir de matière première comprenant au moins un élément du groupe constitué par un dispositif de commande de niveau, une sonde de température et une sonde de pression.

10. Système selon l'une quelconque des revendications précédentes, le bioréacteur comprenant au moins une sonde du groupe constitué par une sonde de pH, une sonde de température, une sonde de pression, une sonde de CO₂ et une sonde de CH₄.

11. Système selon l'une quelconque des revendications précédentes, le système étant de taille de laboratoire ou de petite taille d'échelle pilote.

12. Procédé de commande d'un système de processus de fermentation biologique selon l'une quelconque des revendications précédentes, ledit procédé comprenant l'utilisation d'une unité informatique au moyen de
- la réception de données sur le poids du contenu dans le bioréacteur ;
- le maintien de l'équilibre de masse dans le bioréacteur en commandant l'ouverture et la fermeture de la vanne d'alimentation et de la vanne de décharge par rapport à une valeur de point de consigne de la quantité de masse sur l'unité informatique.
